# EUROPÄISCHE PATENTSCHRIFT

(11) **EP 2 735 259 B1**
(45) Veröffentlichungstag und Bekanntmachung des Hinweises auf die Patenterteilung: **23.03.2022**
(21) Anmeldenummer: 13187132.9
(22) Anmeldetag: 02.10.2013
(51) Int. Cl.: A61B 1/00

(54) **Ein Endoskop mit einstellbarer Blickrichtung**
Endoscope with adjustable view direction
Endoscope à direction d'observation réglable

(30) Priorität: 22.11.2012 DE 102012111290
(43) Veröffentlichungstag der Anmeldung: 28.05.2014
(73) Patentinhaber: Karl Storz SE & Co. KG, 78532 Tuttlingen (DE)
(72) Erfinder: Dahmen, Jan, 78606 Seitingen (DE)

(56) Entgegenhaltungen:
- EP-A1- 2 353 493
- EP-A2- 2 430 971
- DE-A1- 10 136 998
- DE-A1-102011 012 426
- DE-B3-102009 049 143
- US-A1- 2010 030 031

## Beschreibung

Die vorliegende Erfindung ist auf ein Endoskop mit einstellbarer Blickrichtung und dabei insbesondere auf die Kopplung zwischen einer Betätigungseinrichtung und der Einstellung der Blickrichtung gerichtet.

Neben Endoskopen mit fester Blickrichtung parallel oder in einem Winkel zur Längsachse des Schafts werden zunehmend Endoskope mit einstellbarer Blickrichtung verwendet. Eine freie Einstellbarkeit der Blickrichtung innerhalb eines vorbestimmten Winkelbereichs kann eine wesentlich flexiblere Anwendung ermöglichen und damit medizinischem Personal ein schnelleres, entspannteres und damit auch sichereres und ökonomischeres Arbeiten ermöglichen.

Um ein Autoklavieren eines Endoskops und damit seine mehrfache medizinische Verwendung zu ermöglichen, muss der Strahlengang des Endoskops durch eine hermetisch dichte Hülle gegen das Eindringen von Feuchtigkeit auch bei hoher Temperatur und Überdruck geschützt sein. Zur Übertragung von an einer Betätigungseinrichtung außerhalb der hermetisch dichten Hülle erzeugten Kräften und Bewegungen in das Innere der hermetisch dichten Hülle wurden mehrere Konzepte entwickelt, die auf einer magnetischen Kopplung beruhen zwischen Magneten außerhalb und innerhalb der hermetisch dichten Hülle beruhen.

In EP 0 907 900 B1 (erstmals veröffentlicht als WO 98/44376 A1) ist ein Endoskop mit einer hermetisch dichten Hülle beschrieben. Eine Bewegung einer Betätigungseinrichtung wird magnetisch von einem außerhalb der Hülle angeordneten Ring auf einen innerhalb der Hülle angeordneten Ring übertragen. Sowohl der äußere Ring als auch der innere Ring sind nicht axial verschiebbar. Eine Rotation des inneren Rings wird mittels eines Gewindegangs auf ein axial verschiebbares Bauelement übertragen.

In EP 2 353 493 A1 ist ein medizinisches Instrument mit einem magnetischen Stellantrieb beschrieben. Je ein oder mehrere Magneten außerhalb und innerhalb einer hermetisch dichten Hülle sind sowohl axial verschiebbar als auch um eine gemeinsame Achse rotierbar, um sowohl axiale Kräfte als auch Drehmomente durch magnetische Kopplung zu übertragen. Axiale Kräfte sind zur Fokussierung oder für andere Vorgänge übertragbar (Absatz [0019]).

In EP 2 430 970 A2 und EP 2 430 971 A2 sind Endoskope mit einstellbarer Blickrichtung beschrieben. Eine Drehbewegung eines Betätigungselements wird mittels eines Getriebes in eine axiale Bewegung eines Zugrohrs umgesetzt.

DE 10 2011 012 426 A1 ist ein endoskopisches Instrument zur Einhandbedienung beschrieben. Ein Stellelement ist an der Außenseite des Instruments so angeordnet, dass es ergonomisch mit einem Finger einer das Instrument haltenden Hand verschiebbar ist, um eine Blickrichtung des endoskopischen Instruments einzustellen (Absätze [0009], [0036], [0051]).

In US 2010/0030031 A1 ist ein Schwenkprismenendoskop gemäß des Oberbegriffs des Anspruchs 1 vorgestellt, das wiederum ein Stellelement an der Außenseite seines Handgriffs aufweist, dessen Drehung über eine axiale Längsbewegung in eine Schwenkbewegung des distalen optischen Bauteils umgesetzt wird.

Eine Aufgabe der vorliegenden Erfindung besteht darin, ein verbessertes Endoskop mit einstellbarer Blickrichtung zu schaffen.

Diese Aufgabe wird durch den Gegenstand des unabhängigen Anspruchs 1 gelöst.

Weiterbildungen sind in den abhängigen Ansprüchen angegeben.

Ein Endoskop mit einstellbarer Blickrichtung umfasst einen Schaft, eine Bildübertragungseinrichtung zum Übertragen eines Bilds von einem distalen Ende des Endoskops zu einem proximalen Ende des Endoskops, eine hermetisch dichte Hülle um die Bildübertragungseinrichtung mit einem Lichteintrittsfenster am distalen Ende des Endoskops, eine Handhabungseinrichtung am proximalen Ende des Endoskops, eine Schwenkbetätigungseinrichtung an der Handhabungseinrichtung zum manuellen Einstellen eines Winkels zwischen der Blickrichtung und einer Längsachse des Schafts, eine Magnetkopplungseinrichtung zum magnetischen Übertragen einer Kraft parallel zur Längsachse des Schafts zwischen der Schwenkbetätigungseinrichtung und einer Kraftübertragungseinrichtung innerhalb der hermetisch dichten Hülle und eine Drehbetätigungseinrichtung an der Handhabungseinrichtung zum manuellen Rotieren der Blickrichtung um die Längsachse des Schafts relativ zur Handhabungseinrichtung.

Das Endoskop ist insbesondere ein medizinisches Endoskop bzw. ein Endoskop für mikroinvasive diagnostische und chirurgische Eingriffe oder für andere medizinische Eingriffe. Mit einer einstellbaren Blickrichtung ist eine Blickrichtung gemeint, die nicht nur um die Längsachse des Schafts des Endoskops rotierbar, sondern deren Winkel zur Längsachse des Schafts ferner einstellbar ist. Der Schaft des Endoskops ist insbesondere starr und insbesondere gerade. Die Bildübertragungseinrichtung umfasst insbesondere im Fall eines geraden und starren Schafts ein Stablinsensystem aus einer Mehrzahl von Stablinsen. Im Fall eines gekrümmten oder krümmbaren Schafts kann die Bildübertragungseinrichtung ein geordnetes Bündel von Lichtleitfasern umfassen. Alternativ kann die Bildübertragungseinrichtung zur elektronischen Übertragung eines Bilds ausgebildet sein, wobei von einem Objekt ausgehendes Licht von einer Kamera am oder nahe dem distalen Ende des Schafts in ein elektronisches Signal gewandelt wird, das das erfasste Bild darstellt und zum proximalen Ende des Endoskops übertragen wird.

Bestandteil der hermetisch dichten Hülle ist insbesondere ein Rohr, das sich vom distalen Ende des Schafts bis zur Handhabungseinrichtung erstreckt, und das insbesondere innerhalb eines Außenschaftrohrs angeordnet ist. Das Außenschaftrohr kann neben diesem Rohr, das Teil der hermetisch dichten Hülle ist, Lichtleiter zur Übertragung von Beleuchtungslicht vom proximalen Ende zum distalen Ende des Endoskops umfassen. Alternativ oder zusätzlich kann das Außenschaftrohr einen oder mehrere Kanäle zum Aufnehmen von einem oder mehreren medizinischen Instrumenten, zum Leiten eines Spülfluids und zum Absaugen von Fluiden und/oder Gewebestücken oder anderen Partikeln umfassen.

Das Lichteintrittsfenster der hermetisch dichten Hülle am distalen Ende des Endoskops ist durch ein hermetisch dicht in das Lichteintrittsfenster gefügtes Fensterbauteil aus einem optisch transparenten Material verschlossen. Insbesondere wenn die Bildübertragungseinrichtung zum optischen Übertragen eines Bilds ausgebildet ist, umfasst die hermetisch dichte Hülle ferner ein Lichtaustrittsfenster, das durch ein entsprechendes Fensterbauteil aus einem optisch transparenten Material verschlossen ist, am proximalen Ende des Endoskops. Sowohl das Lichteintrittsfenster als auch gegebenenfalls das Lichtaustrittsfenster können jeweils eben oder gekrümmt und ferner als Linsen oder Prismen ausgebildet sein.

Die Handhabungseinrichtung weist insbesondere die Gestalt eines ergonomisch vorteilhaft ausgestalteten Griffs auf. Die Schwenkbetätigungseinrichtung und die Drehbetätigungseinrichtung sind insbesondere so an der Handhabungseinrichtung angeordnet, dass sie jeweils mit einem oder mehreren Fingern einer die Handhabungseinrichtung greifenden und haltenden Hand ohne Weiteres betätigt werden können.

Erfindungsgemäß ist die Kraftübertragungseinrichtung innerhalb der hermetisch dichten Hülle ausgebildet, um eine mittels der Schwenkbetätigungseinrichtung manuell erzeugte Kraft und eine entsprechende Bewegung zum Einstellen des Winkels zwischen Blickrichtung und Längsachse des Schafts zum distalen Ende des Endoskops zu übertragen. Am distalen Ende des Endoskops ist die Kraftübertragungseinrichtung insbesondere mit einem schwenkbaren oder anderweitig bewegbaren Spiegel oder Prisma oder einer anderen optischen Einrichtung zum Einstellen der Blickrichtung mechanisch gekoppelt.

Die Drehbetätigungseinrichtung ist insbesondere zum manuellen Rotieren der Bildübertragungseinrichtung und insbesondere auch zum manuellen Rotieren der hermetisch dichten Hülle um die Längsachse des Schafts ausgebildet.

Die Ausgestaltung der Magnetkopplungseinrichtung zum magnetischen Übertragen einer Kraft parallel zur Längsachse des Schafts kann eine unerwartet einfache Konstruktion und Fertigung ermöglichen. Dazu kann ferner beitragen, dass die Drehbetätigungseinrichtung rein mechanisch ausgebildet sein kann und die Magnetkopplungseinrichtung lediglich zum Übertragen einer Kraft zwischen der Schwenkbetätigungseinrichtung und der Kraftübertragungseinrichtung ausgebildet sein kann.

Bei einem Endoskop, wie es hier beschrieben ist, ist insbesondere die Kraftübertragungseinrichtung parallel zur Längsachse des Schafts verschiebbar und erstreckt sich von der Magnetkopplungseinrichtung bis zum distalen Ende des Endoskops.

Bei einem Endoskop, wie es hier beschrieben ist, ist die Magnetkopplungseinrichtung insbesondere ausgebildet, um kein Drehmoment zu übertragen.

Insbesondere ist die Magnetkopplungseinrichtung ausgebildet, um kein Drehmoment um die Längsachse des Schafts zu übertragen. Dazu weist die Magnetkopplungseinrichtung insbesondere ein durch einen oder mehrere Permanentmagneten erzeugtes Magnetfeld auf, das rotationssymmetrisch zur Längsachse des Schafts ist.

Die Ausbildung der Magnetkopplungseinrichtung derart, dass sie kein Drehmoment überträgt, kann ermöglichen, dass die Kraftübertragungseinrichtung und mit ihr insbesondere auch die Bildübertragungseinrichtung ohne eine Rückwirkung auf den Winkel zwischen Blickrichtung und Längsachse des Schafts um die Längsachse des Schafts rotiert werden kann. Dies kann ermöglichen, dass ein Schwenken der Blickrichtung (Verändern des Winkels zwischen Blickrichtung und Längsachse des Schafts) und ein Rotieren der Blickrichtung um die Längsachse des Schafts völlig unabhängig voneinander erfolgen können. Dabei hat insbesondere weder eine Betätigung der Schwenkbetätigungseinrichtung eine Rotation der Blickrichtung um die Längsachse des Schafts noch eine Betätigung der Drehbetätigungseinrichtung eine Veränderung des Winkels zwischen Blickrichtung und Längsachse des Schafts zur Folge. Allein durch die Ausgestaltung der Magnetkopplungseinrichtung derart, dass sie kein Drehmoment überträgt, kann eine besonders einfache Handhabung des Endoskops ermöglicht werden.

Bei einem Endoskop, wie es hier beschrieben ist, ist die Schwenkbetätigungseinrichtung insbesondere mittels eines Fingers einer die Handhabungseinrichtung greifenden Hand entlang eines vorbestimmten Pfads verschiebbar.

Die Schwenkbetätigungseinrichtung ist insbesondere entlang eines geraden und insbesondere zur Längsachse des Schafts parallelen Pfads verschiebbar. Die Schwenkbetätigungseinrichtung ist an der Handhabungseinrichtung ergonomisch vorteilhaft angeordnet. Dazu ist die Schwenkbetätigungseinrichtung insbesondere derart an der Handhabungseinrichtung angeordnet, dass beim Ergreifen der Handhabungseinrichtung mit einer Hand ohne Weiteres und ohne eine von einem natürlichen Bewegungsablauf abweichende Bewegung ein Finger an der Schwenkbetätigungseinrichtung zu liegen kommt.

Bei einem Endoskop, wie es hier beschrieben ist, ist die Drehbetätigungseinrichtung insbesondere am distalen Ende der Handhabungseinrichtung angeordnet.

Die Drehbetätigungseinrichtung ist insbesondere im Übergangsbereich zwischen Handhabungseinrichtung und Schaft angeordnet. Diese Anordnung kann ergonomisch vorteilhaft sein, insbesondere ein manuelles Rotieren der Blickrichtung um die Längsachse des Schafts mittels eines oder zweier Finger einer die Handhabungseinrichtung greifenden und haltenden Hand ermöglichen.

Bei einem Endoskop, wie es hier beschrieben ist, ist die Drehbetätigungseinrichtung insbesondere mit einem proximalen Bereich eines Außenschaftrohrs starr verbunden.

Die Drehbetätigungseinrichtung weist insbesondere im Wesentlichen die Gestalt eines Rads oder Rings auf, dessen äußerer Umfang geriffelt, profiliert oder auf andere Weise so gestaltet sein kann, dass zur Betätigung nicht allein ein Kraftschluss zwischen den betätigenden Fingern und der Drehbetätigungseinrichtung erforderlich ist, sondern auch Formschluss ohne Weiteres herstellbar ist. Die Ausgestaltung der Drehbetätigungseinrichtung als Rad oder Ring kann eine besonders intuitive Bedienung, insbesondere eine intuitive Rotation der Blickrichtung um die Längsachse des Schafts, ermöglichen.

Ein erfindungsgemäßes Endoskop, wie es hier beschrieben ist, umfasst ferner ein Getriebe zum Übertragen einer ersten Kraft und eines ersten Wegs an der Schwenkbetätigungseinrichtung in eine zweite Kraft und einen zweiten Weg an der Kraftübertragungseinrichtung, wobei erfindungsgemäß die Kraftübertragungseinrichtung parallel zur Längsachse des Schafts verschiebbar ist und sich von der Handhabungseinrichtung bis zum distalen Ende des Endoskops erstreckt.

Das Getriebe umfasst insbesondere einen oder mehrere Hebel oder eine oder mehrere in radialer Richtung bewegbare Kugeln zwischen konischen Flächen unterschiedlicher Steigungen an An- und Abtrieb des Getriebes. Das Getriebe ist erfindungsgemäß ein Untersetzungsgetriebe zum Übertragen einer kleineren Kraft und eines längeren Wegs an der Schwenkbetätigungseinrichtung in eine größere Kraft und einen kürzeren Weg an der Kraftübertragungseinrichtung. Das Getriebe ist erfindungsgemäß zwischen der Magnetkopplungseinrichtung und der Kraftübertragungseinrichtung angeordnet und mit diesen jeweils teilweise integriert.

Die zum Verstellen des Winkels zwischen Blickrichtung und Längsachse des Schafts erforderlichen Wege der Kraftübertragungseinrichtung sind in der Regel im Verhältnis zu der an einer Betätigungseinrichtung manuell erzeugbaren Bewegung kurz. Die Anordnung eines Untersetzungsgetriebes zwischen der Schwenkbetätigungseinrichtung und der Kraftübertragungseinrichtung kann eine feinfühligere manuelle Einstellung des Winkels zwischen Blickrichtung und Längsachse des Schafts vereinfachen.

Bei einem Endoskop, wie es hier beschrieben ist, umfasst insbesondere die Magnetkopplungseinrichtung ein ringförmiges Bauteil mit einer rotationssymmetrischen Innenfläche, deren Durchmesser in einer Richtung parallel zur Längsachse des Schafts kontinuierlich zunimmt, innerhalb der hermetisch dichten Hülle.

An dem ringförmigen Bauteil innerhalb der hermetisch dichten Hülle ist insbesondere eine rotationssymmetrische Anordnung von einem oder mehreren Magneten bzw. eine Anordnung von einem oder mehreren Magneten, die ein zur Längsachse des Schafts rotationssymmetrisches Magnetfeld erzeugen, vorgesehen. Die rotationssymmetrische Innenfläche ist insbesondere konisch bzw. weist die Gestalt einer Mantelfläche eines Kreiskegelstumpfs oder eines anderen Ausschnitts einer Mantelfläche eines Kreiskegels auf.

Bei einem Endoskop, wie es hier beschrieben ist, ist das ringförmige Bauteil insbesondere parallel zur Längsachse des Schafts bewegbar und um die Längsachse des Schafts rotierbar.

Die Rotierbarkeit des ringförmigen Bauteils um die Längsachse des Schafts kann eine alternative oder zusätzliche Maßnahme zur bereits in anderem Zusammenhang beschriebenen Entkopplung des Schwenkens und des Rotierens der Blickrichtung bilden und so einen Beitrag zur intuitiven und ökonomischen Handhabung des Endoskops leisten.

Bei einem Endoskop, wie es hier beschrieben ist, bewirkt insbesondere eine Rotation des ringförmigen Bauteils keine Änderung des Winkels zwischen Blickrichtung und Längsachse des Schafts und keine Rotation der Blickrichtung um die Längsachse des Schafts.

Bei einem Endoskop, wie es hier beschrieben ist, ist die Kraftübertragungseinrichtung insbesondere rohrförmig und umgibt die Bildübertragungseinrichtung im Wesentlichen mantelförmig.

Abweichungen der Kraftübertragungseinrichtung von einer rohrförmigen bzw. zylindermantelförmigen (insbesondere kreiszylindermantelförmigen) Gestalt können insbesondere in einer oder mehreren Öffnungen bzw. Ausnehmungen bestehen. Weitere Abweichungen von einer ideal rohrförmigen bzw. zylindermantelförmigen Gestalt bestehen insbesondere an den Enden der Kraftübertragungseinrichtung. Insbesondere an diesen Enden kann die Kraftübertragungseinrichtung ferner ausgebildet sein, um die Bildübertragungseinrichtung nicht mantelförmig zu umgeben.

Bei einem Endoskop, wie es hier beschrieben ist, ist die Kraftübertragungseinrichtung insbesondere durch eine Einrichtung formschlüssig an einer Rotation relativ zur Bildübertragungseinrichtung gehindert.

Die Einrichtung zum Hindern der Kraftübertragungseinrichtung an einer Rotation relativ zur Bildübertragungseinrichtung umfasst insbesondere einen Stift, der in ein Langloch eingreift, wobei der Stift an der Kraftübertragungseinrichtung und das Langloch an der Bildübertragungseinrichtung oder an der hermetisch dichten Hülle vorgesehen sein kann oder umgekehrt.

Bei einem Endoskop, wie es hier beschrieben ist, ist das Außenschaftrohr insbesondere mit der hermetisch dichten Hülle gekoppelt.

Insbesondere sind Außenschaftrohr und die hermetisch dichte Hülle mechanisch starr miteinander verbunden. Die mechanisch starre Verbindung oder andersartige mechanische Kopplung zwischen Außenschaftrohr und hermetisch dichter Hülle ist insbesondere zwischen den distalen Enden des Außenschaftrohrs und der hermetisch dichten Hülle vorgesehen. In diesem Fall kann proximal der distalen Enden von Außenschaftrohr und hermetisch dichter Hülle ein Zwischenraum vorgesehen sein. In diesem Zwischenraum können ein oder mehrere Lichtleiter zum Übertragen von Beleuchtungslicht und/oder ein oder mehrere Kanäle zum Aufnehmen medizinischer Instrumente, zum Leiten eines Spülfluids und zum Absaugen von Fluiden und Partikeln vorgesehen sein.

Bei einem Endoskop, wie es hier beschrieben ist, können die hermetisch dichte Hülle und die Bildübertragungseinrichtung mechanisch starr miteinander verbunden oder auf andere Weise mechanisch gekoppelt sein. Die hermetisch dichte Hülle und die Bildübertragungseinrichtung sind insbesondere am distalen Ende des Endoskops und/oder im Bereich der Handhabungseinrichtung miteinander starr verbunden. Dazwischen kann ein mantelförmiger (insbesondere kreiszylindermantelförmiger oder anders zylindermantelförmiger) Zwischenraum vorgesehen sein, in dem die Kraftübertragungseinrichtung angeordnet ist.

Ein Endoskop, wie es hier beschrieben ist, umfasst insbesondere ferner eine von außen ablesbare Anzeige des momentan eingestellten Winkels zwischen Blickrichtung und Längsachse des Schafts.

Die von außen ablesbare Anzeige ist insbesondere von außen sichtbar und damit visuell ablesbar. Die Anzeige kann eine Skala und einen Zeiger oder ein Fenster zur Sichtbarmachung von einem oder mehreren Zahlen oder anderen Indikatoren der Skala umfassen.

Ein Endoskop, wie es hier beschrieben ist, ist insbesondere so ausgebildet, dass eine Betätigung der Drehbetätigungseinrichtung keinen Einfluss auf den eingestellten Winkel zwischen der Blickrichtung und der Längsachse des Schafts hat.

### Kurzbeschreibung der Figuren

Nachfolgend werden Ausführungsformen anhand der beigefügten Figuren näher erläutert. Es zeigen:
- Figur 1: eine schematische Darstellung eines Endoskops mit einstellbarer Blickrichtung;
- Figur 2: eine schematische Schnittdarstellung eines proximalen Bereichs einer Ausführungsform des Endoskops aus Figur 1;
- Figur 3: eine weitere schematische Schnittdarstellung des proximalen Bereichs der Ausführungsform aus Figur 2;
- Figur 4: eine schematische Schnittdarstellung eines proximalen Bereichs einer weiteren Ausführungsform des Endoskops aus Figur 1;
- Figur 5: eine weitere schematische Schnittdarstellung des proximalen Bereichs der Ausführungsform aus Figur 4.

### Beschreibung der Ausführungsformen

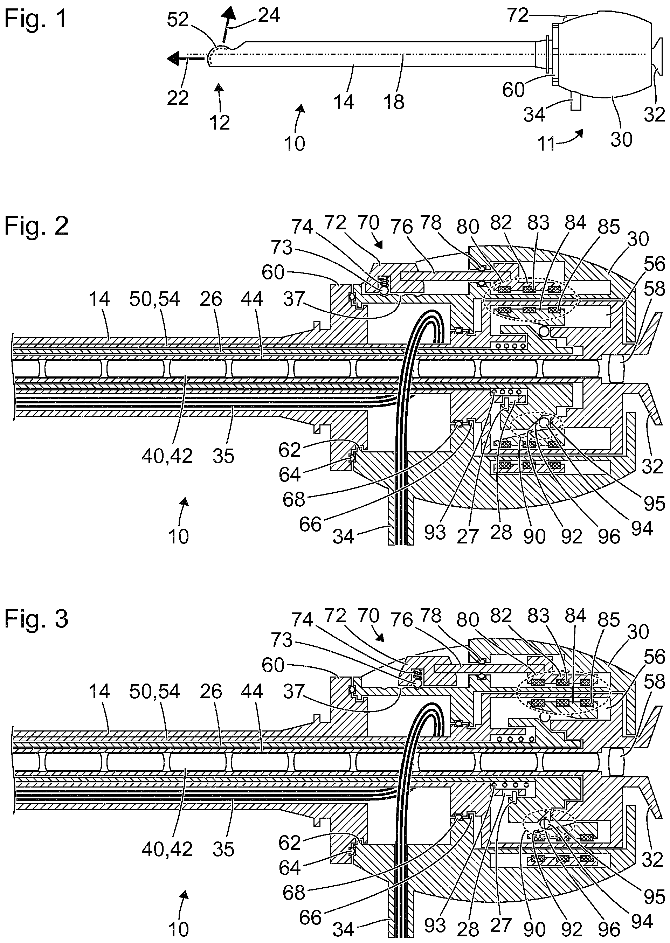

Figur 1 zeigt eine schematische Darstellung eines Endoskops 10 mit einem proximalen Ende 11 und einem distalen Ende 12. Ein Schaft, von dem nur ein Außenschaftrohr 14 mit einer Längsachse 18 sichtbar ist, erstreckt sich vom proximalen Ende 11 bis zum distalen Ende 12 des Endoskops 10. Am distalen Ende 12 des Endoskops sind zwei extreme Blickrichtungen 22, 24 angedeutet, zwischen denen die Blickrichtung (insbesondere stufenlos) einstellbar ist.

Am proximalen Ende 11 des Endoskops 10 ist eine Handhabungseinrichtung 30 mit einem Okular 32 und einer Kupplung 34 für ein Lichtleitkabel vorgesehen. Über die Kupplung 34 ist die Handhabungseinrichtung 30 mittels eines in den Figuren nicht dargestellten Lichtleitkabels mit einer Lichtquelle zum Bereitstellen von Beleuchtungslicht koppelbar.

An der Handhabungseinrichtung 30 sind ferner eine Drehbetätigungseinrichtung 60 und eine Schwenkbetätigungseinrichtung 70 vorgesehen. Die Drehbetätigungseinrichtung 60 ist im Übergangsbereich zwischen der Handhabungseinrichtung 30 und dem Schaft 14 bzw. am distalen Ende der Handhabungseinrichtung 30 und am proximalen Ende des Schafts 14 angeordnet. Die Drehbetätigungseinrichtung 60 weist die Gestalt eines profilierten Rads auf, das mittels eines oder mehrerer Finger einer die Handhabungseinrichtung 30 greifenden Hand um die Längsachse 18 des Schafts 14 gedreht werden kann, um die Blickrichtung des Endoskops 10 um die Längsachse 18 des Schafts 14 zu rotieren. Die Schwenkbetätigungseinrichtung 70 ist so an der Handhabungseinrichtung 30 angeordnet, dass sie mittels eines Fingers einer die Handhabungseinrichtung 30 greifenden und haltenden Hand entlang eines Pfads parallel zur Längsachse 18 des Schafts 14 verschiebbar ist, um den Winkel zwischen der Blickrichtung 22, 24 und der Längsachse 18 des Schafts 14 zu verändern.

Am distalen Ende 12 des Endoskops 10 ist ein Lichteintrittsfenster 52 angedeutet, durch das Licht von einem in der momentan eingestellten Blickrichtung 22, 24 liegenden Gegenstand in den Schaft 14 eintreten kann. Nachfolgend wird nicht zwischen dem Lichteintrittsfenster 52 bzw. der entsprechenden Öffnung einerseits und dem das Lichteintrittsfenster 52 verschließenden Fensterbauteil aus einem optisch transparenten Material unterschieden. Das Lichteintrittsfenster 52 weist insbesondere die Gestalt eines Ausschnitts eines Kreiszylindermantels mit Zylinderachse senkrecht zur Zeichenebene der Figur 1 oder die Gestalt eines Ausschnitts einer Kugeloberfläche oder einer Oberfläche eines Ellipsoids auf.

Figur 2 zeigt eine schematische Schnittdarstellung eines proximalen Bereichs einer Ausführungsform des Endoskops 10 aus Figur 1. Die Schnittebene der Figur 2 ist parallel zur Zeichenebene der Figur 1 und enthält die Längsachse 18 (vgl. Figur 1) des Schafts 14.

Im Außenschaftrohr 14 des Endoskops 10 sind nebeneinander ein Innenschaftrohr 54 und ein Bündel Lichtleiter 35 angeordnet. Die Lichtleiter 35 führen von der Kupplung 34 an der Handhabungseinrichtung 30 zum distalen Ende 12 des Endoskops 10 (vgl. Figur 1). Mittels eines in den Figuren nicht dargestellten Lichtleitkabels von einer Lichtquelle zur Kupplung 34 an der Handhabungseinrichtung 30 übertragenes Beleuchtungslicht kann von den Lichtleitern 35 zum distalen Ende 12 des Endoskops 10 übertragen werden, dort austreten und einen zu beobachtenden Gegenstand beleuchten. Zwischen der Kupplung 34 und dem Außenschaftrohr 14 sind die Lichtleiter 35 in einem Hohlraum so geführt, dass das Außenschaftrohr 14 innerhalb eines vorbestimmten Winkelbereichs um seine Längsachse 18 (vgl. Figur 1) relativ zur Handhabungseinrichtung 30 rotiert werden kann.

Das Innenschaftrohr 54 ist Bestandteil einer hermetisch dichten Hülle 50. Die hermetisch dichte Hülle 50 weist in einem Bereich innerhalb der Handhabungseinrichtung 30 einen deutlich vergrößerten Querschnitt auf und umschließt dort eine Kammer 56, die unten beschrieben ist. Am distalen Ende 12 des Endoskops 10 weist die hermetisch dichte Hülle 50 das oben anhand der Figur 1 dargestellte Lichteintrittsfenster 52 mit einem das Lichteintrittsfenster 52 hermetisch dicht verschließenden Fensterbauteil aus einem optisch transparenten Material auf. Am proximalen Ende 11 des Endoskops 10 weist die hermetisch dichte Hülle 50 ein Lichtaustrittsfenster 58 und ein das Lichtaustrittsfenster 58 hermetisch dicht verschließendes Fensterbauteil aus einem optisch transparenten Material auf. Das Lichtaustrittsfenster 58 ist insbesondere als Linse ausgebildet und Bestandteil des Okulars 32 oder nahe dem Okular 32 angeordnet.

Die hermetisch dichte Hülle 50 umschließt bzw. enthält insbesondere im Bereich des Innenschaftrohrs 54, aber auch im Bereich der Kammer 56, eine Bildübertragungseinrichtung 40 mit einer Mehrzahl von Stablinsen 42 in einem Systemschaft 44. Ferner umschließt die hermetisch dichte Hülle 50 ein Objektiv bzw. eine oder mehrere Linsen oder Spiegel sowie einen schwenkbaren Spiegel, ein schwenkbares Prisma oder eine andere optische Einrichtung zum Einstellen der Blickrichtung 22, 24 (vgl. Figur 1), die in den Figuren nicht dargestellt sind, am distalen Ende 12 des Endoskops 10.

Zwischen dem Innenschaftrohr 54 und dem Systemschaft 44 befindet sich ein im Wesentlichen zylindermantelförmiger Zwischenraum, in dem ein Schub- und Zugrohr als Kraftübertragungseinrichtung 26 angeordnet ist. Die Kraftübertragungseinrichtung 26 ist parallel zur Längsachse 18 (vgl. Figur 1) des Außenschaftrohrs 14 bewegbar. Die Kraftübertragungseinrichtung 26 ist am distalen Ende 12 des Endoskops 10 mit der erwähnten Einrichtung zum Einstellen der Blickrichtung (insbesondere schwenkbarer Spiegel oder schwenkbares Prisma) mechanisch gekoppelt. Durch Verschiebung der Kraftübertragungseinrichtung 26 parallel zur Längsachse 18 des Außenschaftrohrs 14 wird die Blickrichtung 22, 24 des Endoskops 10 verstellt bzw. verändert.

Die bereits oben anhand der Figur 1 dargestellte Drehbetätigungseinrichtung 60 ist mit dem proximalen Ende des Außenschaftrohrs 14 einstückig ausgebildet oder stoff-, kraft- und/oder formschlüssig gefügt. Zwischen der Drehbetätigungseinrichtung 60 und der Handhabungseinrichtung 30 sind ein erstes, distales Rotationslager 62, das in Figur 2 als einfaches Gleitlager angedeutet ist, und eine erste, distale Dichtung 64 vorgesehen. Proximal des ersten Rotationslagers 62 und der ersten Dichtung 64 und in Richtung parallel zur Längsachse 18 des Außenschaftrohrs 14 von diesen beabstandet sind ein zweites, proximales Rotationslager 66 und eine zweite, proximale Dichtung 68 zwischen dem mit dem Außenschaftrohr 14 starr verbundenen Innenschaftrohr 54 einerseits und der Handhabungseinrichtung 30 andererseits vorgesehen. Die Rotationslager 62, 66 ermöglichen eine Rotation der Drehbetätigungseinrichtung 60 zusammen mit dem Außenschaftrohr 14, der hermetisch dichten Hülle 50 mit den darin angeordneten Einrichtungen und der Lichtleiter 35 (soweit sie im Bereich des Außenschaftrohrs 14 angeordnet sind) um die Längsachse 18 (vgl. Figur 1) des Außenschaftrohrs 14. Die Dichtungen 64, 68 verhindern weitgehend oder vollständig ein Eindringen von Fluiden und eine dadurch hervorgerufene Beschädigung der Lichtleiter 35.

Die bereits oben in Zusammenhang mit Figur 1 erwähnte Schwenkbetätigungseinrichtung 70 umfasst einen Schiebegriff 72, der in einer Ausnehmung an der Außenseite der Handhabungseinrichtung 30 parallel zur Längsachse 18 angeordnet ist. Der Schiebegriff 72 ist so angeordnet, dass er durch einen Finger einer Hand, die die Handhabungseinrichtung 30 greift und hält, parallel zur Längsachse 18 (vgl. Figur 1) verschiebbar ist.

Der Schiebegriff 72 umfasst eine Rastkugel 73, die durch eine Rastfeder 74 gegen eine Oberfläche der Handhabungseinrichtung 30 gedrückt wird. In der genannten Oberfläche der Handhabungseinrichtung 30 sind mehrere Rastnuten 37 vorgesehen, in die die Rastkugel 73 eingreifen kann, um den Schiebegriff 72 in korrespondierenden Positionen zu halten.

Der Schiebegriff 72 ist durch eine Schubstange 76 mit einer Magnetkopplungseinrichtung 80 im Inneren der Handhabungseinrichtung 30 mechanisch gekoppelt, insbesondere starr verbunden. An der Schubstange 76 ist eine Dichtung 78 vorgesehen, um ein Eindringen von Fluiden in das Innere der Handhabungseinrichtung 30 und insbesondere zur Magnetkopplungseinrichtung 80 zu verhindern.

Die Magnetkopplungseinrichtung 80 umfasst einen äußeren Magnetträger 82 mit einer Mehrzahl von Magneten 83 außerhalb der hermetisch dichten Hülle 50 und insbesondere außerhalb der durch die hermetisch dichte Hülle 50 umschlossenen Kammer 56. Ferner umfasst die Magnetkopplungseinrichtung 80 einen inneren Magnetträger 84 mit einer Mehrzahl von Magneten 85 innerhalb der durch die hermetisch dichte Hülle 50 umschlossenen Kammer 56. Der äußere Magnetträger 82 und der innere Magnetträger 84 sind jeweils insbesondere im Wesentlichen ringförmig ausgebildet. Eine Abweichung von einer idealen Ringform ergibt sich am äußeren Magnetträger 82 insbesondere durch die mechanische Verbindung mit dem proximalen Ende der Schubstange 76. Sowohl der äußere Magnetträger 82 als auch der innere Magnetträger 84 sind jeweils parallel zur Längsachse 18 (vgl. Figur 1) bewegbar spiel- und reibungsarm geführt.

Die Magneten 83 am äußeren Magnetträger 82 und die Magneten 85 am inneren Magnetträger 84 sind jeweils ausgebildet, um zur Längsachse 18 (vgl. Figur 1) rotationssymmetrische Magnetfelder zu erzeugen. Insbesondere sind sowohl die Magneten 83 am äußeren Magnetträger 82 als auch die Magneten 85 am inneren Magnetträger 84 jeweils kreisringförmig. Alternativ erzeugen lediglich entweder die Magneten 83 am äußeren Magnetträger 82 oder die Magneten 85 am inneren Magnetträger 84 jeweils ein zur Längsachse 18 rotationssymmetrisches Magnetfeld. Dadurch ist kein Drehmoment zwischen den Magneten 83 am äußeren Magnetträger 82 und den Magneten 85 am inneren Magnetträger 84 übertragbar.

Die Magneten 83 am äußeren Magnetträger 82 und die Magneten 85 am inneren Magnetträger 84 sind jeweils so polarisiert, dass anziehende und abstoßende Kräfte zwischen den Magneten 83 am äußeren Magnetträger 82 einerseits und den Magneten 85 am inneren Magnetträger 84 andererseits die in Figur 2 angedeutete relative Anordnung der Magnetträger 82, 84 bewirken. Eine Verschiebung des inneren Magnetträgers 84 gegenüber dem äußeren Magnetträger 82 in Richtung parallel zur Längsachse 18 erfordert eine mit zunehmender Auslenkung stark ansteigende Kraft. Aufgrund der spiel- und reibungsarmen Lagerung sowohl des äußeren Magnetträgers 82 außerhalb der hermetisch dichten Hülle 50 als auch des inneren Magnetträgers 84 innerhalb der Kammer 56 in der hermetisch dichten Hülle 50 können jedoch der äußere Magnetträger 82 und der innere Magnetträger 84 gemeinsam parallel zur Längsachse 18 bewegt werden. Insbesondere bewirkt eine manuell hervorgerufene Bewegung des Schiebegriffs 72 vermittels der Schubstange 76 eine entsprechende Bewegung des äußeren Magnetträgers 82 und vermittels der magnetischen Kopplung zwischen den Magneten 83 am äußeren Magnetträger 82 einerseits und den Magneten 85 am inneren Magnetträger 84 andererseits eine entsprechende Bewegung des inneren Magnetträgers 84.

Der innere Magnetträger 84 ist gleichzeitig Bestandteil eines Getriebes 90 zum Übertragen, insbesondere Untersetzen, einer Bewegung an der Schwenkbetätigungseinrichtung 70 und der Magnetkopplungseinrichtung 80 einerseits in eine Bewegung der Kraftübertragungseinrichtung 26 andererseits, wobei die wirkenden Kräfte und die zurückgelegten Wege an der Schwenkbetätigungseinrichtung 70 und der Magnetkopplungseinrichtung 80 einerseits sich von denen an der Kraftübertragungseinrichtung 26 unterscheiden.

Das Getriebe 90 umfasst einen Innenkonus 92 am inneren Magnetträger 84, einen Außenkonus 96 am proximalen Ende der Kraftübertragungseinrichtung 26, Kugeln 94 zwischen dem Innenkonus 92 am inneren Magnetträger 84 und dem Außenkonus 96 an der Kraftübertragungseinrichtung 26, eine Führung 95 für die Kugeln 94 und eine Druckfeder 93 zwischen der hermetisch dichten Hülle 50 einerseits und der Kraftübertragungseinrichtung 26 andererseits.

Der Innenkonus 92 am inneren Magnetträger 84 und der Außenkonus 96 am proximalen Ende der Kraftübertragungseinrichtung 26 weisen jeweils eine konstante Steigung und die Gestalt eines ringförmigen Ausschnitts eines Kegelmantels auf. Die Steigungen bzw. die Öffnungswinkel des Innenkonus 92 am inneren Magnetträger 84 und des Außenkonus 96 am proximalen Ende der Kraftübertragungseinrichtung 26 unterscheiden sich voneinander. Die Führung 95 für die Kugeln 94 wird durch eine ringförmige und ebene Anlagefläche senkrecht zur Längsachse 18 des Außenschaftrohrs 14 (vgl. Figur 1) gebildet. Die Druckfeder 93 schiebt die Kraftübertragungseinrichtung 26 nach proximal, so dass jederzeit der Außenkonus 96 am proximalen Ende der Kraftübertragungseinrichtung 26 an den Kugeln 94 und die Kugeln 94 an der Führung 95 und am Innenkonus 92 am inneren Magnetträger 84 anliegen.

Eine Bewegung des inneren Magnetträgers 84 in Richtung parallel zur Längsachse 18 (vgl. Figur 1) des Außenschaftrohrs 14 bewirkt eine Bewegung der Kugeln 94 in radialer Richtung entlang der Führung 95 und eine Bewegung der Kraftübertragungseinrichtung 26 in Richtung parallel zur Längsachse 18 des Außenschaftrohrs 14. Figur 3 zeigt eine weitere schematische Schnittdarstellung des in Figur 2 gezeigten proximalen Bereichs der Ausführungsform des Endoskops 10 in einer weiteren Konfiguration bzw. Situation. Die Schwenkbetätigungseinrichtung 70, die Magnetkopplungseinrichtung 80 und mit ihr der Innenkonus 92 am inneren Magnetträger 84 einerseits und die Kraftübertragungseinrichtung 26 andererseits befinden sich in der in Figur 2 dargestellten Situation jeweils in einer distalen Position und in der in Figur 3 dargestellten Situation jeweils in einer proximalen Position.

Die unterschiedlichen Steigungen bzw. Öffnungswinkel des Innenkonus 92 am inneren Magnetträger 84 einerseits und des Außenkonus 96 am proximalen Ende der Kraftübertragungseinrichtung 26 andererseits bewirken eine Untersetzung bzw. eine Übertragung eines längeren Wegs an der Schwenkbetätigungseinrichtung 70 und der Magnetkopplungseinrichtung 80 einerseits in einen kürzeren Weg an der Kraftübertragungseinrichtung 26 andererseits. Entsprechend wird eine kleinere Kraft an der Schwenkbetätigungseinrichtung 70 und an der Magnetkopplungseinrichtung 80 einerseits in eine größere Kraft an der Kraftübertragungseinrichtung 26 übertragen.

Abweichend von der Darstellung in den Figuren 2 und 3 können die Fläche am proximalen Ende der Kraftübertragungseinrichtung 26 und/oder die Fläche am inneren Magnetträger 84, an denen die Kugeln 94 anliegen, nicht-konisch ausgebildet sein bzw. eine in Richtung parallel zur Längsachse 18 (vgl. Figur 1) des Außenschaftrohrs 14 variierende Steigung aufweisen. Ferner kann abweichend von der Darstellung in den Figuren 2 und 3 die Führung 95 für die Kugeln 94 nicht eben, sondern gekrümmt (beispielsweise konisch) ausgebildet sein. Dadurch kann ein über den Weg variierendes Untersetzungsverhältnis eingestellt werden.

Am proximalen Ende der Kraftübertragungseinrichtung 26 ist ein Führungsstift 27 vorgesehen, der in einen Führungsschlitz 28 eingreift. Der Führungsschlitz 28 ist mit der hermetisch dichten Hülle 50 und über diese mit der Bildübertragungseinrichtung 40 starr verbunden. Der Führungsstift 27 ist im Führungsschlitz 28 spiel- und reibungsarm in Richtung parallel zur Längsachse (vgl. Figur 1) des Außenschaftrohrs 14 bewegbar geführt. Der Führungsstift 27 und der Führungsschlitz 28 bilden eine Einrichtung zum formschlüssigen Hindern der Kraftübertragungseinrichtung 26 an einer Rotation relativ zur Bildübertragungseinrichtung 40. Diese Einrichtung schützt die mechanische Kopplung zwischen der Kraftübertragungseinrichtung 26 und einem schwenkbaren Spiegel oder Prisma oder einer anderen die Blickrichtung 22, 24 (vgl. Figur 1) bestimmenden Einrichtung am distalen Ende 12 des Endoskops 10 vor einer Torsionsbelastung.

Bei einer mittels der Drehbetätigungseinrichtung 60 manuell bewirkten Rotation des Außenschaftrohrs 14 zusammen mit der hermetisch dichten Hülle 50, der Kraftübertragungseinrichtung 26 und der Bildübertragungseinrichtung 40 um die Längsachse 18 (vgl. Figur 1) des Außenschaftrohrs 14 werden der Außenkonus 96 am proximalen Ende der Kraftübertragungseinrichtung 26 und die Führung 95 einerseits gegenüber dem äußeren Magnetträger 82 rotiert. Die beschriebene Ausgestaltung der Magneten 83 am äußeren Magnetträger 82 und der Magneten 85 am inneren Magnetträger 84 derart, dass die erzeugten Magnetfelder rotationssymmetrisch zur Längsachse 18 des Außenschaftrohrs 14 sind, verhindert dabei ein die Rotation hemmendes oder behinderndes Drehmoment. Dieses hemmende oder behindernde Drehmoment ist alternativ oder zusätzlich durch eine freie Rotierbarkeit des inneren Magnetträgers 84 um die Längsachse 18 des Außenschaftrohrs 14 (bis auf mechanische Reibung zwischen Außenkonus 96, Kugeln 94, Führung 95 und Innenkonus 92) vermeidbar. Dies trägt dazu bei, dass ein Verändern des Winkels zwischen Blickrichtung 22, 24 und Längsachse 18 des Außenschaftrohrs 14 mittels der Schwenkbetätigungseinrichtung 70 einerseits und ein Rotieren der Blickrichtung 22, 24 um die Längsachse 18 des Außenschaftrohrs 14 mittels der Drehbetätigungseinrichtung 60 andererseits unabhängig voneinander und ohne gegenseitige Beeinflussung erfolgen können.

Bei der Ausführungsform der Figuren 2 und 3 sind also mehrere Maßnahmen verwirklicht, die eine freie Rotierbarkeit des Außenschaftrohrs 14 mit der hermetisch dichten Hülle 50 und der Bildübertragungseinrichtung 40 einerseits gegenüber der Handhabungseinrichtung 30 andererseits ohne Rückwirkung auf den Winkel zwischen Blickrichtung 22, 24 (vgl. Figur 1) und Längsachse 18 des Außenschaftrohrs 14 ermöglichen. Alternativ kann lediglich eine oder zwei der Maßnahmen realisiert sein, also entweder die Rotationssymmetrie des durch die Magneten 83 am äußeren Magnetträger 82 erzeugten Magnetfelds oder die Rotationssymmetrie des durch die Magneten 85 am inneren Magnetträger 84 erzeugten Magnetfelds oder die Rotationssymmetrie des Innenkonus 92 am inneren Magnetträger 84, der Führung 95 und des Außenkonus 96 am proximalen Ende der Kraftübertragungseinrichtung 26.

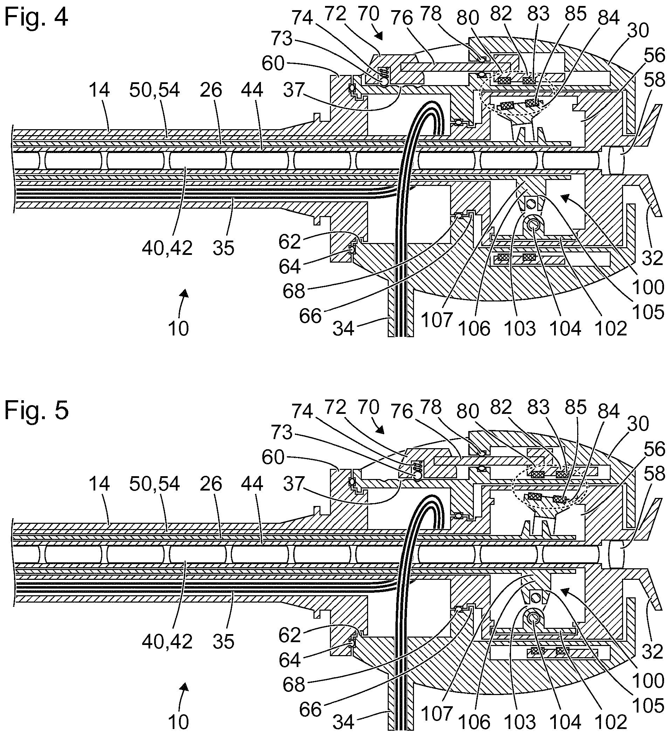

Figur 4 zeigt eine schematische Schnittdarstellung eines proximalen Bereichs einer weiteren Ausführungsform des Endoskops 10 aus Figur 1. Die Art der Darstellung und insbesondere die Schnittebene parallel zur Längsachse 18 (vgl. Figur 1) des Außenschaftrohrs 14 entsprechen denen der Darstellungen in den Figuren 2 und 3. Die Ausführungsform der Figur 4 ähnelt in einigen Merkmalen, Eigenschaften und Funktionen der Ausführungsformen der Figuren 2 und 3. Nachfolgend sind lediglich Merkmale, Eigenschaften und Funktionen dargestellt, in denen sich die Ausführungsform der Figur 4 von der Ausführungsform der Figur 2 und 3 unterscheidet.

Die Ausführungsform der Figur 4 unterscheidet sich von der Ausführungsform der Figuren 2 und 3 insbesondere dadurch, dass anstelle eines Getriebes mit Kugeln zwischen einem Innenkonus und einem Außenkonus mit unterschiedlichen Steigungen ein Getriebe 100 mit einem Hebel 103 vorgesehen ist. Der Hebel 103 ist mittels eines Schwenkgelenks 104 mit einem Schlitten 102 gekoppelt. Der Schlitten 102 ist in der Kammer 56 innerhalb der hermetisch dichten Hülle 50 frei um die Längsachse 18 (vgl. Figur 1) des Außenschaftrohrs 14 rotierbar spiel- und reibungsarm formschlüssig geführt. Das Schwenkgelenk 104 definiert eine Schwenkachse, die senkrecht zur Längsachse 18 des Außenschaftrohrs 14 und bei der in Figur 4 dargestellten Position des Schlittens 102 senkrecht zur Schnittebene der Figur 4 ist.

Am vom Schwenkgelenk 104 abgewandten Ende weist der Hebel 103 einen inneren Magnetträger 84 mit mehreren Magneten 85 auf. Im Unterschied zur Ausführungsform der Figuren 2 und 3 ist der innere Magnetträger 84 nicht parallel zur Längsachse 18 des Außenschaftrohrs 14 verschiebbar, sondern um die durch das Schwenkgelenk 104 definierte Schwenkachse schwenkbar. Die Magneten 83 am äußeren Magnetträger 82 und die Magneten 85 am inneren Magnetträger 84 sind so angeordnet und ausgebildet, insbesondere polarisiert, dass jede lineare Translationsbewegung des äußeren Magnetträgers 82 eine korrespondierende Schwenkbewegung des inneren Magnetträgers 84 und damit des Hebels 103 um die durch das Schwenkgelenk 104 definierte Schwenkachse bewirkt.

Der Hebel 103 weist eine Öffnung 105 auf, in die das proximale Ende der Kraftübertragungseinrichtung 26 eingreift. Insbesondere umschließt der Hebel 103 die Öffnung 105 rahmenförmig. Innerhalb der Öffnung 105 ist ein Stift 106 parallel zu der durch das Schwenkgelenk 104 definierten Schwenkachse vorgesehen. Der Stift 106 greift in eine umlaufende Nut 107 an der Kraftübertragungseinrichtung 26 ein. Der Stift 106 am Hebel 103 und die umlaufende Nut 107 an der Kraftübertragungseinrichtung 26 sind so ausgebildet, dass sie eine spiel- und reibungsarme, formschlüssige mechanische Kopplung des Hebels 103 mit der Kraftübertragungseinrichtung 26 für Kräfte parallel zur Längsachse 18 (vgl. Figur 1) des Außenschaftrohrs 14 ermöglichen und gleichzeitig eine Rotation des Hebels 103 (zusammen mit dem Schlitten 102) um die Längsachse 18 des Außenschaftrohrs 14 relativ zur Kraftübertragungseinrichtung 26 nicht behindern oder hemmen.

Abweichend von der Darstellung in Figur 4 kann ähnlich wie bei der Ausführungsform der Figuren 2 und 3 ein Führungsstift in einem Führungsschlitz oder eine andere Einrichtung zum formschlüssigen Hindern der Kraftübertragungseinrichtung 26 an einer Rotation relativ zur Bildübertragungseinrichtung 40 vorgesehen sein.

Eine Bewegung der Schwenkbetätigungseinrichtung 70 und vermittels der Schubstange 76 der Magnetkopplungseinrichtung 80 bewirkt eine korrespondierende Schwenkbewegung 103 und aufgrund der formschlüssigen Kopplung zwischen dem Stift 106 am Hebel 103 und der umlaufenden Nut 107 an der Kraftübertragungseinrichtung 26 eine Bewegung der Kraftübertragungseinrichtung 26 parallel zur Längsachse 18 (vgl. Figur 1) des Außenschaftrohrs 14.

Figur 5 zeigt eine weitere schematische Darstellung des proximalen Bereichs der Ausführungsform aus Figur 4 des Endoskops 10. Die Art der Darstellung, insbesondere die Schnittebene, entspricht denen der Figuren 2 bis 4. Die in Figur 5 dargestellte Situation unterscheidet von der in Figur 4 dargestellten dadurch, dass die Schwenkbetätigungseinrichtung 70 und die Magnetkopplungseinrichtung 80 einerseits und mit diesen vermittels des Getriebes 100 auch die Kraftübertragungseinrichtung 26 nach proximal verschoben sind.

Das Verhältnis des Abstands des Stifts 106 von der durch das Schwenkgelenk 104 definierten Schwenkachse und des Abstands der Magnetkopplungseinrichtung 80 von der durch das Schwenkgelenk 104 definierten Schwenkachse bewirkt eine entsprechende Untersetzung bzw. eine Übertragung eines längeren Wegs und einer kleineren Kraft an der Schwenkbetätigungseinrichtung 70 und der Magnetkopplungseinrichtung 80 in einen kürzeren Weg und eine größere Kraft an der Kraftübertragungseinrichtung 26.

Die Magneten 83 am äußeren Magnetträger 82 sind insbesondere so angeordnet und polarisiert, dass sie ein zur Längsachse 18 (vgl. Figur 1) des Außenschaftrohrs 14 rotationssymmetrisches Magnetfeld erzeugen. Diese Ausgestaltung verhindert ein Drehmoment, das eine Rotation des Hebels 103 (zusammen mit dem Schlitten 102) um die Längsachse 18 bewirkt, hemmt oder behindert. Ein derartiges hemmendes oder hinderndes Drehmoment ist zusätzlich durch die beschriebene freie Rotierbarkeit des Hebels 103 zusammen mit dem inneren Magnetträger 84 und dem Schlitten 102 um die Längsachse 18 des Außenschaftrohrs 14 (bis auf Reibung zwischen Schlitten 102 und hermetisch dichter Hülle 50) vermeidbar. Dies trägt dazu bei, dass ein Verändern des Winkels zwischen Blickrichtung 22, 24 und Längsachse 18 des Außenschaftrohrs 14 mittels der Schwenkbetätigungseinrichtung 70 einerseits und ein Rotieren der Blickrichtung 22, 24 um die Längsachse 18 des Außenschaftrohrs 14 mittels der Drehbetätigungseinrichtung 60 andererseits unabhängig voneinander und ohne gegenseitige Beeinflussung erfolgen können.

Bei der Ausführungsform der Figuren 4 und 5 sind also zwei Maßnahmen verwirklicht, die eine freie Rotierbarkeit des Außenschaftrohrs 14 mit der hermetisch dichten Hülle 50 und der Bildübertragungseinrichtung 40 einerseits gegenüber der Handhabungseinrichtung 30 andererseits ohne Rückwirkung auf den Winkel zwischen Blickrichtung 22, 24 (vgl. Figur 1) und Längsachse 18 des Außenschaftrohrs 14 ermöglichen. Alternativ kann lediglich eine der beiden Maßnahmen realisiert sein, also entweder die Rotationssymmetrie des durch die Magneten 83 am äußeren Magnetträger 82 erzeugten Magnetfelds oder die Rotierbarkeit des Hebels 103 mit dem Schlitten 102 um die Längsachse 18.

Bei dem anhand der Figur 1 dargestellten Endoskop 10 und insbesondere bei jeder der anhand der Figuren 2 bis 5 dargestellten Ausführungsformen kann eine in den Figuren nicht dargestellte Anzeige des momentan eingestellten Winkels vorgesehen sein. Insbesondere sind an einer äußeren Oberfläche der Handhabungseinrichtung 30 eine Skala mit Zahlen oder anderen Symbolen, die den Wert des Winkels zwischen Blickrichtung 22, 24 (vgl. Figur 1) und Längsachse 18 des Außenschaftrohrs 14 darstellen, und am Schiebegriff 72 oder mit diesem mechanisch gekoppelt ein Zeiger, der auf die Skala zeigt, vorgesehen. Alternativ kann ein Fenster am Schiebegriff 72 oder mit diesem mechanisch gekoppelt vorgesehen sein, das einen Wert oder ein Symbol an der Skala hervorhebt. Alternativ können die Skala am Schiebegriff 72 oder mit diesem mechanisch gekoppelt und der Zeiger, das Fenster oder eine andere Einrichtung zum sichtbaren Hervorheben am Gehäuse der Handhabungseinrichtung 30 vorgesehen sein.

### Bezugszeichen

- **10**: **Endoskop**
- 11: proximales Ende des Endoskops 10
- 12: distales Ende des Endoskops 10
- 14: Schaft, Außenschaftrohr des Endoskops 10
- 18: Längsachse des Schafts 14
- 22: erste extreme Blickrichtung des Endoskops 10
- 24: zweite extreme Blickrichtung des Endoskops 10
- 26: Kraftübertragungseinrichtung bzw. Schub- und Zugrohr
- 27: Führungsstift am proximalen Ende der Kraftübertragungseinrichtung 26
- 28: Führungsschlitz an der hermetisch dichten Hülle 50
- **30**: **Handhabungseinrichtung am proximalen Ende 11 des Endoskops 10**
- 32: Okular an der Handhabungseinrichtung 20
- 34: Kupplung für Lichtleitkabel
- 35: Lichtleiter im Außenschaftrohr 14
- 37: Rastnut an Handhabungseinrichtung 20 für Rastkugel 73
- **40**: **Bildübertragungseinrichtung**
- 42: Stablinse der Bildübertragungseinrichtung 40
- 44: Systemschaft der Bildübertragungseinrichtung 40
- **50**: **hermetisch dichte Hülle 50**
- 52: Lichteintrittsfenster am distalen Ende der hermetisch dichten Hülle 50
- 54: Innenschaftrohr der hermetisch dichten Hülle 50
- 56: Kammer der hermetisch dichten Hülle 50
- 58: Lichtaustrittsfenster am distalen Ende der hermetisch dichten Hülle 50
- **60**: **Drehbetätigungseinrichtung an der Handhabungseinrichtung 20**
- 62: erstes (distales) Rotationslager zwischen Schaft 14 und Handhabungseinrichtung 30
- 64: erste (distale) Dichtung zwischen Schaft 14 und Handhabungseinrichtung 30
- 66: zweites (proximales) Rotationslager zwischen Schaft 14 und Handhabungseinrichtung 30
- 68: zweite (proximale) Dichtung zwischen Schaft 14 und Handhabungseinrichtung 30
- **70**: **Schwenkbetätigungseinrichtung an der Handhabungseinrichtung 20**
- 72: Schiebegriff der Schwenkbetätigungseinrichtung 70
- 73: Rastkugel am Schiebegriff 72
- 74: Rastfeder am Schiebegriff 72
- 76: Schubstange
- 78: Dichtung an der Schubstange 76
- **80**: **Magnetkopplungseinrichtung**
- 82: äußerer Magnetträger der Magnetkopplungseinrichtung 80
- 83: Magnet am äußeren Magnetträger 82
- 84: innerer Magnetträger der Magnetkopplungseinrichtung 80
- 85: Magnet am inneren Magnetträger 84
- **90**: **Getriebe**
- 92: Innenkonus am inneren Magnetträger 84
- 93: Druckfeder zwischen Hülle 50 und Kraftübertragungseinrichtung 26
- 94: Kugel zwischen Innenkonus 94 und Außenkonus 96
- 95: Führung für Kugel 94
- 96: Außenkonus am proximalen Ende der Kraftübertragungseinrichtung 26
- **100**: **Getriebe**
- 102: Schlitten
- 103: Hebel
- 104: Schwenkgelenk zwischen Schlitten 102 und Hebel 103
- 105: Öffnung im Hebel 103
- 106: Stift am Hebel 103
- 107: umlaufende Nut an der Kraftübertragungseinrichtung 26

## Patentansprüche

1. **Endoskop** (10) mit einstellbarer Blickrichtung (22, 24), mit:
einem **Schaft** (14);
einer **Bildübertragungseinrichtung** (40) eingerichtet zum Übertragen eines Bilds von einem
distalen Ende (12) des Endoskops (10) zu einem proximalen Ende (11) des Endoskops (10);
einer hermetisch dichten **Hülle** (50) um die Bildübertragungseinrichtung (42, 44),
mit einem Lichteintrittsfenster (52) am distalen Ende (12) des Endoskops (10);
einer **Handhabungseinrichtung** (30) am proximalen Ende (11) des Endoskops (10);
einer **Schwenkbetätigungseinrichtung** (70) an der Handhabungseinrichtung (30), eingerichtet zum manuellen Einstellen eines Winkels zwischen der Blickrichtung (22, 24) und einer Längsachse (18) des Schafts (14);
einer Kraftübertragungseinrichtung (26) innerhalb der hermetisch dichten Hülle (50), wobei die Kraftübertragungseinrichtung (26) parallel zur Längsachse (18) des Schafts (14) verschiebbar ist und sich von der Handhabungseinrichtung (30) bis zum distalen Ende (12) des Endoskops (10) erstreckt , und wobei die Kraftübertragungseinrichtung (26) eingerichtet ist zum Übertragen einer mittels der Schwenkbetätigungseinrichtung (70) manuell erzeugten Kraft und einer entsprechenden Bewegung zum distalen Ende (12) des Endoskops (10) zum Einstellen des Winkels zwischen Blickrichtung (22, 24) und Längsachse (18) des Schafts (14), wobei die Kraftübertragungseinrichtung (26) am distalen Ende (12) des Endoskops (10) mit einem schwenkbaren oder anderweitig bewegbaren Spiegel, Prisma oder einer anderen optischen Einrichtung zum Einstellen der Blickrichtung mechanisch gekoppelt ist;
einer **Magnetkopplungseinrichtung** (80) eingerichtet zum magnetischen Übertragen einer
**Kraft parallel zur Längsachse** (18) des Schafts (14) zwischen der Schwenkbetätigungseinrichtung (70) und der **Kraftübertragungseinrichtung** (26) innerhalb der hermetisch dichten Hülle (50);
einer **Drehbetätigungseinrichtung** (60) an der Handhabungseinrichtung (30), eingerichtet zum
manuellen Rotieren der Blickrichtung (22, 24) um die Längsachse (18) des Schafts (14) relativ zur Handhabungseinrichtung (20);
einem **Getriebe** (90; 100) eingerichtet zum Übertragen einer ersten Kraft und eines ersten Wegs an der Schwenkbetätigungseinrichtung (70) in eine zweite Kraft und einen zweiten Weg an der Kraftübertragungseinrichtung (26),
**dadurch gekennzeichnet, dass**
das Getriebe (90; 100) als ein Untersetzungsgetriebe ausgebildet ist zum Übertragen einer kleineren Kraft und eines längeren Wegs an der Schwenkbetätigungseinrichtung (70) in eine größere Kraft und einen kürzeren Weg an der Kraftübertragungseinrichtung (26),
und dadurch, dass das Getriebe (90; 100) zwischen der Magnetkopplungseinrichtung (80) und der Kraftübertragungseinrichtung (26) angeordnet und mit diesen jeweils teilweise integriert ist.

2. Endoskop (10) nach dem vorangehenden Anspruch, bei dem die **Drehbetätigungseinrichtung** (60) **am distalen Ende** der Handhabungseinrichtung (30) angeordnet ist.

3. Endoskop (10) nach einem der vorangehenden Ansprüche, bei dem die **Drehbetätigungseinrichtung** (60) mit einem proximalen Bereich eines **Außenschaftrohrs** (14) **starr verbunden** ist.

4. Endoskop (10) nach einem der vorangehenden Ansprüche, bei dem die Magnetkopplungseinrichtung (80) ein **ringförmiges Bauteil** (84) mit einer rotationssymmetrischen Innenfläche (92), deren **Durchmesser** in einer Richtung parallel zur Längsachse (18) des Schafts (14) kontinuierlich **zunimmt,** innerhalb der hermetisch dichten Hülle (50) umfasst.

5. Endoskop (10) nach dem vorangehenden Anspruch, bei dem das **ringförmige Bauteil** (84) parallel zur Längsachse (18) des Schafts (14) **bewegbar** und um die Längsachse (18) des Schafts (14) **rotierbar** ist.

6. Endoskop (10) nach einem der vorangehenden Ansprüche, bei dem die **Kraftübertragungseinrichtung** (26) **rohrförmig** ist und die Bildübertragungseinrichtung (40) im Wesentlichen mantelförmig umgibt.

7. Endoskop (10) nach dem vorangehenden Anspruch, bei dem die Kraftübertragungseinrichtung (26) durch eine Einrichtung (27, 28) formschlüssig an einer Rotation relativ zur Bildübertragungseinrichtung (40) gehindert ist.

8. Endoskop (10) nach dem vorangehenden Anspruch, bei dem das **Außenschaftrohr** (14) mit der hermetisch dichten **Hülle** (40) **gekoppelt** ist.

9. Endoskop (10) nach einem der vorangehenden Ansprüche, ferner mit:
einer von außen ablesbaren **Anzeige des** momentan eingestellten **Winkels** zwischen Blickrichtung (22, 24) und Längsachse (18) des Schafts (14).

## Claims

1. Endoscope (10) with an adjustable viewing direction (22, 24), comprising:
a shaft (14);
an image transfer device (40) configured to transfer an image from a distal end (12) of the endoscope (10) to a proximal end (11) of the endoscope (10);
a hermetically sealed sleeve (50) around the image transfer device (42, 44), having a light entrance window (52) at the distal end (12) of the endoscope (10);
a handling device (30) at the proximal end (11) of the endoscope (10);
a pivot actuation device (70) located at the handling device (30) and configured to manually adjust an angle between the viewing direction (22, 24) and a longitudinal axis (18) of the shaft (14);
a force transfer device (26) within the hermetically sealed sleeve (50), the force transfer device (26) being displaceable parallel to the longitudinal axis (18) of the shaft (14) and extending from the handling device (30) to the distal end (12) of the endoscope (10), and the force transfer device (26) being configured to transfer a force generated manually by means of the pivot actuation device (70) and a corresponding movement to the distal end (12) of the endoscope (10) for the purposes of adjusting the angle between the viewing direction (22, 24) and the longitudinal axis (18) and the shaft (14), the force transfer device (26) being mechanically coupled at the distal end (12) of the endoscope (10) to a pivotable or otherwise movable mirror, prism or any other optical device for adjusting the viewing direction;
a magnetic coupling device (80) configured for magnetic transfer of a force parallel to the longitudinal axis (18) of the shaft (14) between the pivot actuation device (70) and the force transfer device (26) within the hermetically sealed sleeve (50);
a rotation actuation device (60) at the handling device (30), configured for manual rotation of the viewing direction (22, 24) about the longitudinal axis (18) of the shaft (14) relative to the handling device (20);
a gearing (90; 100) configured to transfer a first force and a first displacement at the pivot actuation device (70) into a second force and a second displacement at the force transfer device (26),
**characterized in that**
the gearing (90; 100) is designed as a reduction gearing for transferring a smaller force and a longer displacement at the pivot actuation device (70) into a greater force and a shorter displacement at the force transfer device (26),
and **in that** the gearing (90; 100) is arranged between the magnetic coupling device (80) and the force transfer device (26) and is at least partly integrated in each of these.

2. Endoscope (10) according to the preceding claim, wherein the rotation actuation device (60) is arranged at the distal end of the handling device (30).

3. Endoscope (10) according to either of the preceding claims, wherein the rotation actuation device (60) is rigidly connected to a proximal region of an outer shaft tube (14).

4. Endoscope (10) according to any one of the preceding claims, wherein the magnetic coupling device (80) comprises within the hermetically sealed sleeve (50) a ring-shaped component (84) with a rotationally symmetric inner surface (92), the diameter of which continuously increases in a direction parallel to the longitudinal axis (18) of the shaft (14).

5. Endoscope (10) according to the preceding claim, wherein the ring-shaped component (84) is movable parallel to the longitudinal axis (18) of the shaft (14) and rotatable about the longitudinal axis (18) of the shaft (14).

6. Endoscope (10) according to any one of the preceding claims, wherein the force transfer device (26) is tubular and surrounds the image transfer device (40) substantially in the form of a lateral surface.

7. Endoscope (10) according to the preceding claim, wherein the force transfer device (26) is interlockingly prevented by way of a device (27, 28) from a rotation relative to the image transfer device (40).

8. Endoscope (10) according to the preceding claim, wherein the outer shaft tube (14) is coupled to the hermetically sealed sleeve (40).

9. Endoscope (10) according to any one of the preceding claims, further comprising:
an externally readable display of the currently set angle between the viewing direction (22, 24) and the longitudinal axis (18) of the shaft (14).

## Revendications

1. Endoscope (10) à direction d'observation réglable (22, 24), ledit endoscope comprenant :
une tige (14) ;
un dispositif de transmission d'image (40) conçu pour transmettre une image d'une extrémité distale (12) de l'endoscope (10) à une extrémité proximale (11) de l'endoscope (10) ;
une gaine hermétiquement étanche (50) disposée autour du dispositif de transmission d'image (42, 44) et pourvue d'une fenêtre d'entrée de lumière (52) à l'extrémité distale (12) de l'endoscope (10) ;
un dispositif de manipulation (30) à l'extrémité proximale (11) de l'endoscope (10) ;
un dispositif d'actionnement pivotant (70) disposé sur le dispositif de manipulation (30) et conçu pour régler manuellement un angle entre la direction d'observation (22, 24) et un axe longitudinal (18) de la tige (14) ;
un dispositif de transmission de force (26) à l'intérieur du boîtier hermétiquement étanche (50), le dispositif de transmission de force (26) pouvant coulisser parallèlement à l'axe longitudinal (18) de la tige (14) et s'étendant du dispositif de manipulation (30) à l'extrémité distale (12) de l'endoscope (10) et le dispositif de transmission de force (26) étant conçu pour transmettre une force générée manuellement au moyen du dispositif d'actionnement pivotant (70) et pour effectuer un déplacement correspondant par rapport à l'extrémité distale (12) de l'endoscope (10) afin de régler l'angle entre la direction d'observation (22, 24) et l'axe longitudinal (18) de la tige (14), le dispositif de transmission de force (26) étant couplé mécaniquement, à l'extrémité distale (12) de l'endoscope (10), à un miroir, un prisme ou un autre dispositif optique pivotant ou déplaçable différemment afin de régler la direction d'observation ;
un dispositif de couplage magnétique (80) conçu pour transmettre magnétiquement une force parallèlement à l'axe longitudinal (18) de la tige (14) entre le dispositif d'actionnement pivotant (70) et le dispositif de transmission de force (26) à l'intérieur de la gaine hermétiquement étanche (50) ;
un dispositif d'actionnement rotatif (60) disposé sur le dispositif de manipulation (30) et conçu pour faire tourner manuellement la direction d'observation (22, 24) sur l'axe longitudinal (18) de la tige (14) par rapport au dispositif de manipulation (20) ;
une transmission (90 ; 100) conçue pour traduire une première force et un premier trajet au niveau du dispositif d'actionnement pivotant (70) en une deuxième force et un deuxième trajet au niveau du dispositif de transmission de force (26),
**caractérisé en ce que**
la transmission (90 ; 100) est conçue comme une transmission de démultiplication destinée à traduire une force plus petite et un trajet plus long au niveau du dispositif d'actionnement pivotant (70) en une force plus grande et un trajet plus court au niveau du dispositif de transmission de force (26), et **en ce que** la transmission (90 ; 100) est disposée entre le dispositif de couplage magnétique (80) et le dispositif de transmission de force (26) et est partiellement intégrée à ceux-ci.

2. Endoscope (10) selon la revendication précédente, dans lequel le dispositif d'actionnement rotatif (60) est disposé à l'extrémité distale du dispositif de manipulation (30).

3. Endoscope (10) selon l'une des revendications précédentes, dans lequel le dispositif d'actionnement rotatif (60) est lié rigidement à une région proximale d'un tube de tige extérieur (14).

4. Endoscope (10) selon l'une des revendications précédentes, dans lequel le dispositif de couplage magnétique (80) est un composant annulaire (84) pourvu d'une surface intérieure (92) à symétrie de révolution dont le diamètre augmente continument dans une direction parallèle à l'axe longitudinal (18) de la tige (14), à l'intérieur de la gaine hermétique étanche (50) .

5. Endoscope (10) selon la revendication précédente, dans lequel le composant annulaire (84) est mobile parallèlement à l'axe longitudinal (18) de la tige (14) et peut tourner sur l'axe longitudinal (18) de la tige (14) .

6. Endoscope (10) selon l'une des revendications précédentes, dans lequel le dispositif de transmission de force (26) est tubulaire et entoure le dispositif de transmission d'image (40) sensiblement sous la forme d'une chemise.

7. Endoscope (10) selon la revendication précédente, dans lequel le dispositif de transmission de force (26) est empêché de tourner par rapport au dispositif de transmission d'image (40) par complémentarité de formes par un dispositif (27, 28).

8. Endoscope (10) selon la revendication précédente, dans lequel le tube de tige extérieur (14) est accouplé à la gaine hermétique étanche (40).

9. Endoscope (10) selon l'une des revendications précédentes, comprenant en outre :
un affichage lisible de l'extérieur de l'angle actuellement réglé entre la direction d'observation (22, 24) et l'axe longitudinal (18) de la tige (14).
